# EUROPEAN PATENT SPECIFICATION

(11) **EP 4 388 996 B1**
(45) Date of publication and mention of the grant of the patent: **22.07.2026**
(21) Application number: 23219356.5
(22) Date of filing: 21.12.2023
(51) Int. Cl.: A61B 5/11, G09B 23/30, G09B 23/32

(54) **A MICRO-VIBRATION TESTING DEVICE FOR VITAL PARAMETER EVALUATION AND A METHOD THEREOF**
MIKROVIBRATIONSPRÜFVORRICHTUNG ZUR VITALPARAMETERBEURTEILUNG UND VERFAHREN DAFÜR
DISPOSITIF DE TEST DE MICRO-VIBRATIONS POUR ÉVALUATION DE PARAMÈTRES VITAUX ET PROCÉDÉ ASSOCIÉ

(30) Priority: 22.12.2022 IN 202241074717
(43) Date of publication of application: 26.06.2024
(73) Proprietor: Turtle Shell Technologies Pvt Ltd, Bengaluru Karnataka 560038 (IN)
(72) Inventor: KAUSHIK, Pavan Kumar, 560038 Bengaluru (IN); KADAMBI, Pooja, 560038 Bengaluru (IN); PARCHANI, Gaurav, 560038 Bengaluru (IN)
(74) Representative: Potter Clarkson

(56) References cited:
- EP-B1- 3 563 368
- US-A1- 2021 127 983
- ANONYMOUS: "Medical patient simulator - Precision Microdrives", 23 October 2021 (2021-10-23), XP093146346, Retrieved from the Internet <URL:https://web.archive.org/web/20211023175415/https://www.precisionmicrodrives.com/case-study/medical-patient-simulator> [retrieved on 20240327]
- SECORD THOMAS ET AL: "A Multi-Actuator Approach to High Bandwidth In Vitro Cardiac Kinematic Simulation", 2018 7TH IEEE INTERNATIONAL CONFERENCE ON BIOMEDICAL ROBOTICS AND BIOMECHATRONICS (BIOROB), IEEE, 26 August 2018 (2018-08-26), pages 833 - 838, XP033417378, DOI: 10.1109/BIOROB.2018.8487781
- KADAMBI POOJA ET AL: "ED.Ai: A Ballistic Simulator for Cardiac and Respiratory Micro-vibration Testing", 2023 15TH INTERNATIONAL CONFERENCE ON COMMUNICATION SYSTEMS & NETWORKS (COMSNETS), IEEE, 3 January 2023 (2023-01-03), pages 448 - 451, XP034296022, DOI: 10.1109/COMSNETS56262.2023.10041349

## Description

### FIELD OF INVENTION

The field of invention generally relates to a micro-vibration validation testing. More specifically, it relates to a system and method for a micro-vibration validation for vital parameter evaluation.

### BACKGROUND

The quantitative evaluation of an individual's health status requires the measurement of physiological parameters. The monitoring of vitals can provide important physiological insights for medical diagnosis and well-being management, making it useful for health monitoring. The vital signs monitoring techniques include optical-based, radar-based, WiFi-based, RFID-based, and acoustic-based methods.

The vital signs monitoring techniques must be validated against a gold-standard reference across a representative patient sample and under representative conditions to gain clinical acceptance.

Typically, the most commonly used method for validating vitals monitors is to use an ECG simulator. However, ECG signals are unsuitable for validating vital monitors that use Ballisto Cardio Graphy (BCG) or Seismography (SG) because they simulate electrical activity rather than the ballistic micro-vibrations required by BCG-based vitals monitors.

Currently, existing systems can simulate micro-vibrations. However, they are mostly used for research purposes and unsuitable for testing vitals monitors. These technologies include shaker tables, pneumatic vibrators, and electrodynamic shakers.

Other existing systems have tried to address this problem. However, their scope was limited to the technologies that are bulky, expensive, and difficult to use. They are also not able to accurately simulate the wide range of cardiac conditions. Other systems also introduce noise vibrations that impact the testing.

Furthermore, the existing systems that are used to validate BCG monitors involve human subjects to artificially increase or decrease their vital parameters. Such an approach not only presents considerable risk to the human subject, but also fails to provide stable signals over a wide range of values.

EP3563368 discloses a heart simulation system for medical services or diagnostic machines.

Thus, in light of the above discussion, it is implied that there is a need for a system and method for a ballistic micro-vibration validation for vital parameters like breathing, heart rate, cardiac output etc. which is reliable and does not suffer from the problems discussed above.

### OBJECT OF INVENTION

The principal object of this invention is to provide a system and method for a ballistic micro-vibration validation.

Another object of the invention is to provide a system and method to validate the vitals monitors.

A further object of the invention is to provide a system and method that can generate micro-vibrations for long durations without putting a human subject at risk, making it a more cost-effective option for monitor validation.

Another object of the invention is to generate micro-vibrations of various heart rates and durations, allowing it to be used to validate a variety of different monitors.

### BRIEF DESCRIPTION OF FIGURES

This invention is illustrated in the accompanying drawings, throughout which, like reference letters, indicate corresponding parts in the various figures.

The embodiments herein will be better understood from the following description with reference to the drawings, in which:
Fig. 1 depicts/illustrates a block diagram of a system for ballistic micro-vibration validation of vitals monitor, in accordance with an embodiment of the present disclosure;
Fig. 2 depicts/illustrates a detailed block diagram of components of the system for ballistic micro-vibration validation of vitals monitor, in accordance with an embodiment of the present disclosure;
Fig. 3a depicts/illustrates a detailed block diagram of components of a heart module of ballistic micro-vibration validation system, in accordance with an embodiment of the present disclosure;
Fig. 3b depicts/illustrates a detailed block diagram of components of a microcontroller module of ballistic micro-vibration validation system, in accordance with an embodiment of the present disclosure;
Fig. 4 depicts/illustrates a flow diagram of ballistic micro-vibration validation of vitals monitor, in accordance with an embodiment of the present disclosure; and
Fig. 5 illustrates a method for ballistic micro-vibration validation of vitals monitor evaluation, in accordance with an embodiment of the present disclosure.
Fig. 6 illustrates a method for ballistic micro-vibration validation of vitals monitor, in accordance with an embodiment of the present disclosure.

### STATEMENT OF INVENTION

The present invention discloses a ballistic micro-vibration validation system for vitals monitor evaluation and a method thereof.

The system comprises at least one user device, at least one vital monitor, at least one ballistic micro-vibration validation device and a communication network.

The ballistic micro-vibration validation device is a versatile and reliable tool for testing vitals monitors and other cardiac devices.

The ballistic micro-vibration validation device comprises a heart module, and a microcontroller module. The heart module comprises a vibration module and the microcontroller module comprises a MOSFET Relay.

The vibration module in the heart module simulates the mechanical micro-vibrations of a heartbeat. The micro-vibrations are controlled by the microcontroller module which drives the vibration module using the MOSFET relay. The system enables a wide range of cardiac states to be simulated, from a healthy heartbeat to arrhythmias.

### DETAILED DESCRIPTION

The embodiments herein and the various features and advantageous details thereof are explained more fully with reference to the non-limiting embodiments that are illustrated in the accompanying drawings and/or detailed in the following description. Descriptions of well-known components and processing techniques are omitted so as to not unnecessarily obscure the embodiments herein. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The present invention discloses a ballistic micro-vibration validation system for a vital monitor. The ballistic micro-vibration system can validate the vital monitor. The ballistic micro-vibration validation system can produce micro-vibrations that are more similar to the natural variability of the human heartbeat. Further, the ballistic micro-vibration validation system can simulate a wide range of micro-vibrations of various heart rates for long durations with high fidelity without putting a human subject at risk, making it a more cost-effective option for monitor validation.

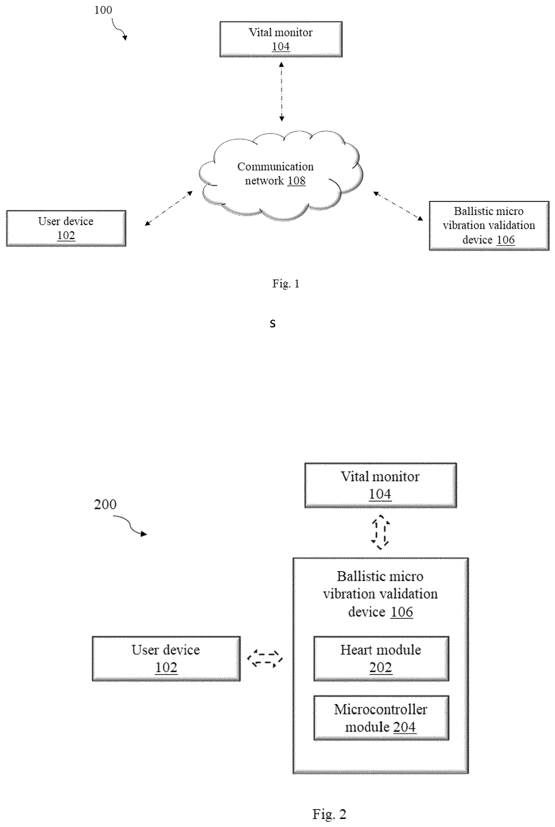

Fig. 1 depicts/illustrates a block diagram of a system for ballistic micro-vibration validation of a vital monitor, in accordance with an embodiment of the present disclosure.

The system 100 comprises at least one user device 102, at least one vital monitor 104, at least one ballistic micro-vibration validation device 106 and a communication network 108.

In an embodiment, the at least one user device 102 may comprise one or more of wearable device, mobile phones, PDA, smartphones, smart band, smart watch, laptop, computer, etc. The user device 102 may have a user application which can control the operations of the system 100.

The at least one vital monitor 104 is configured to evaluate at least one vital sign of at least one patient. The vital sign may comprise at least one of heart rate, blood oxygen saturation, and blood pressure among others. The at least one vital monitor 104 is validated without putting a human subject at risk.

The at least one ballistic micro-vibration validation device 106 is configured to generate at least one micro-vibrations of at least one of various heart rates and durations, enabling it to be used to validate the at least one vital monitor 104.

The at least one vital monitor 104 comprises one or more sensors for measuring at least one of heart rate, blood oxygen saturation, and blood pressure.

Typically, the at least one ballistic micro-vibration validation device 106 is connected to the at least one vital monitor 104 and the at least one user device 102 via the communication network 108. One skilled in the art may recognize that the communication network 108 may be a wired network or wireless network.

Furthermore, the wired communication may be carried out by any one of the network configurations such as LAN, WAN, etc. and the wireless communication may be carried out through Mobile Service Provider (MSP) and Internet Service Provider (ISP) having internet connection provided by an ISP provider, 2G/3G/4G/5G internet connection provided by the mobile service provider. The standard protocols such as TCP/IP, HTTP, FTP, UDP, IPV4, IPV6 etc. as known in the art, may be used for the wireless communication.

Fig. 2 depicts/illustrates a detailed block diagram of components of the system for ballistic micro-vibration validation of vitals monitor, in accordance with an embodiment of the present disclosure.

The ballistic micro-vibration validation device 106 comprises a heart module 202, and a microcontroller module 204. The heart module 202 simulates a mechanical micro-vibration of a heartbeat.

In an embodiment, the heart module 202 simulates heart rates as low as 25 bpm to 200 bpm, a heart rate variability (HRV) of 0ms to 200ms, and arrhythmias such as supraventricular tachycardia, bradycardia etc.

The microcontroller module 204 is configured to precisely control and deliver the power to the heart module 202. The microcontroller module 204 can independently vary heart rate. Further, the microcontroller module 204 can sweep and hold through a huge range of cardiac function states for vital monitor validation and quality assurance.

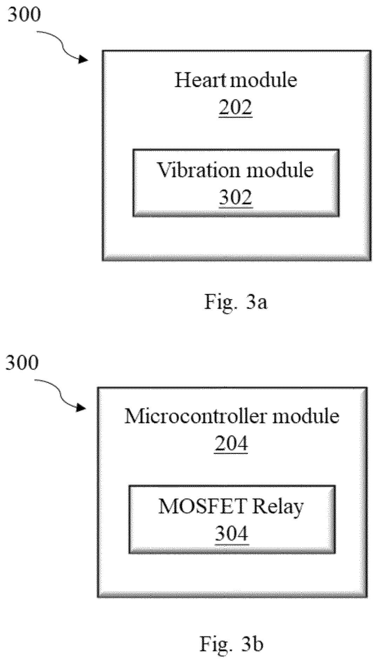

Fig. 3a depicts/illustrates a detailed block diagram of components of a heart module of a ballistic micro-vibration validation system, in accordance with an embodiment of the present disclosure.

In an embodiment, the cardiac cycle (lub-dub) comprises all of the physiological events associated with a single heartbeat. The atria and ventricles alternately contract in each cardiac cycle. Further, the cardiac cycle is split into two phases, systole that corresponds to the contraction phase and diastole that corresponds to the relaxation phase. Therefore, the cardiac cycle includes four stages comprising atrial systole, ventricular systole, atrial diastole, and ventricular diastole.

In an embodiment, the lub represents ventricular systole and diastole whereas dub represents atrial systole and diastole.

Typically, heart sounds are created from blood flowing through the heart chambers as the cardiac valves open and close during the cardiac cycle. The present invention replicates the micro-vibrations of the heart.

The heart module 202 comprises a vibration module 302. According to the invention, the vibration module 302 is an Eccentric Rotating Mass (ERM) which simulates the mechanical micro-vibrations of the heartbeat.

The vibration of the heart is simulated by precise manipulation of the power and timing to the vibration module 302. Two short pulses in rapid succession are used to create a lub-dub signal. The lub-dub signal is repeated for every cycle and the interval is set based on the desired heart rate by the microcontroller module 204. Further, the interval is varied based on the desired heart rate variability (HRV) and arrhythmia condition.

Fig. 3b depicts/illustrates a detailed block diagram of components of a microcontroller module of ballistic micro-vibration validation system, in accordance with an embodiment of the present disclosure.

The microcontroller module 204 comprises a MOSFET Relay 304 which is configured to drive the ERM.

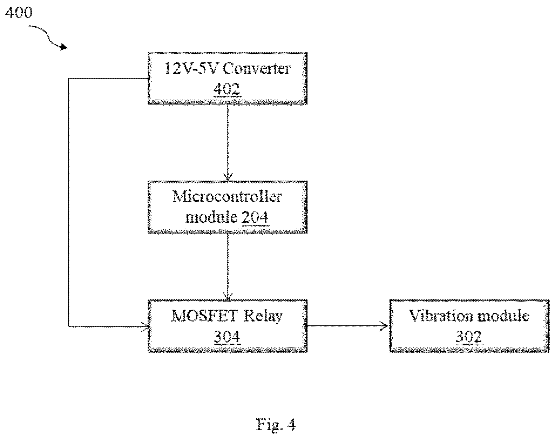

Fig. 4 depicts/illustrates a flow diagram of ballistic micro-vibration validation of vitals monitor, in accordance with an embodiment of the present disclosure;

The flow diagram of ballistic micro-vibration validation of vitals monitor comprises a 12V-5V converter 402, a microcontroller module 204, a MOSFET Relay 304, and a vibration module 302.

The 12V-5V converter 402 is a step down converter which is configured to convert 12 V to 5V as the microcontroller module 204 require 5 V DC supply for its operation.

In an embodiment, the microcontroller module 204 comprises at least one of Arduino UNO, ESP32, and Rpi.

In the preferred embodiment, the microcontroller module 204 is an Arduino UNO. Arduino UNO is a low-cost, flexible, and easy-to-use programmable open-source microcontroller board.

In an embodiment, the MOSFET Relay 304 is an IRF 520 Relay. IRF520 MOSFET is a third-generation power MOSFET that provides fast switching, cost-effectiveness, strong design, and less resistance.

In an embodiment, the vibration module 302 is a vibration motor. With a high number of revolutions per minute, the motor is constantly being displaced and moved by these asymmetric forces, thereby creating micro-vibration by repeated displacement of the motor.

The ballistic micro-vibration validation system 100 is an electromechanical system. In an embodiment, the system 100 comprises the heart module 202 and the microcontroller module 204. The heart module 202 is configured to simulate the micro-vibrations of individual heart beats, whereas the microcontroller module 204 is configured to control at least one of duration and intensity of the micro-vibrations. These two modules work together to enable the system 100 to simulate a wide range of cardiac states, from a healthy heartbeat to arrhythmias. The generation of micro-vibrations of various heart rates and durations that closely match the natural variability of the human heartbeat, facilitates the system 100 to validate the at least one vital monitor 104.

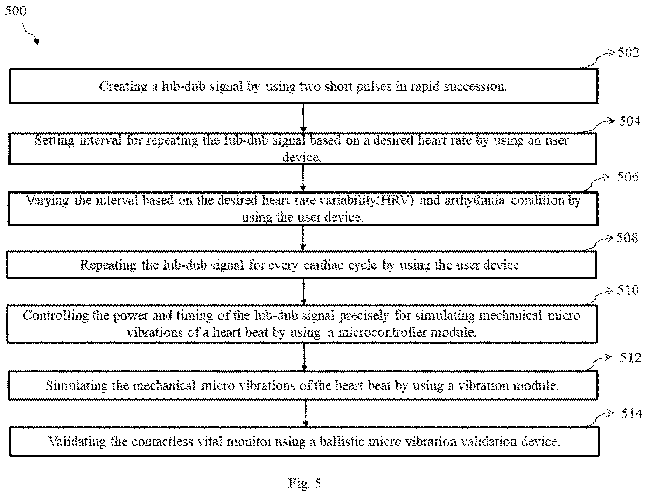

Fig. 5 illustrates a method for ballistic micro-vibration validation of vitals monitor evaluation, in accordance with an embodiment of the present disclosure;

The method begins with creating a lub-dub signal by using two short pulses in rapid succession, as depicted at step 502. Subsequently, the method 500 discloses setting interval for repeating the lub-dub signal based on a desired heart rate by using a user device, as depicted at step 504. Thereafter, the method 500 discloses varying the interval based on the desired heart rate variability (HRV) and arrhythmia condition by using the user device, as depicted at step 506. Subsequently, the method 500 discloses repeating the lub-dub signal for every cardiac cycle by using the user device, as depicted at step 508.

Thereafter, the method 500 discloses controlling the power and timing of the lub-dub signal precisely for simulating mechanical micro-vibrations of a heart beat by using a microcontroller module, as depicted at step 510. Subsequently, the method 500 discloses simulating the mechanical micro-vibrations of the heart beat by using a vibration module, as depicted at step 512. Thereafter, the method 500 discloses validating the vital monitor using a ballistic micro-vibration validation device, as depicted at step 514.

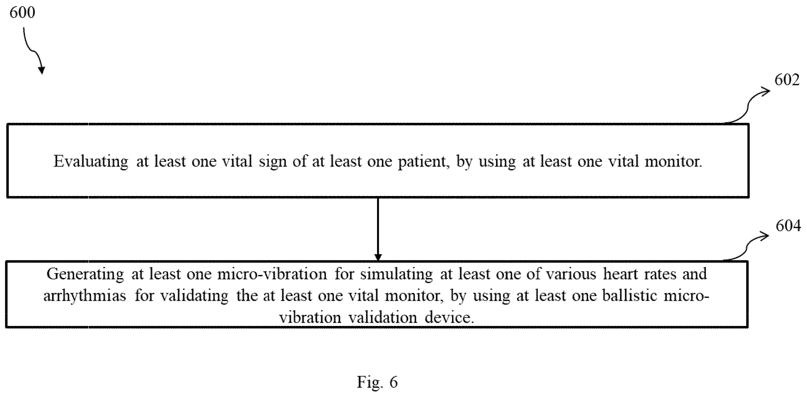

Figure 6 illustrates a method 600 ballistic micro-vibration validation of vitals monitor evaluation. The method 600 begins with evaluating at least one vital sign of at least one patient, by using at least one vital monitor, as depicted at step 602. Subsequently, the method 600 discloses generating at least one micro-vibration for simulating at least one of various heart rates and arrhythmias for validating the at least one vital monitor, by using at least one ballistic micro-vibration validation device, as depicted at step 604.

The advantages of the current invention include validation of the vital monitors.

An additional advantage is that the ballistic micro-vibration validation device can generate micro-vibrations for long durations without putting a human subject at risk, making it a more cost-effective option for monitor validation.

Furthermore, the ballistic micro-vibration validation system is more accurate than other validation systems, as it can reproduce the natural variability of the human heartbeat.

Applications of the current invention include medical device validation and micro-vibration validation.

## Claims

1. A system (100) for ballistic micro-vibration validation of vitals monitor, comprising:
at least one vital monitor (104) configured to evaluate at least one vital sign of at least one patient;
a heart module (202) configured to simulate a mechanical micro-vibration of a heartbeat;
a microcontroller module (204) configured to control and deliver power to the heart module (202); and
at least one ballistic micro-vibration validation device (106) configured to generate at least one micro-vibration for simulating at least one of various heart rates and arrhythmias for validating the at least one vital monitor (104);
**characterized in that** the mechanical micro-vibration is simulated by using at least one eccentric rotating mass (302).

2. The system (100) as claimed in claim 1, wherein the at least one vital monitor (104) comprises one or more sensors for measuring at least one of heart rate, blood oxygen saturation, and blood pressure.

3. The system (100) as claimed in claim 1, wherein the at least one ballistic micro-vibration validation device (106) enables simulation of at least one of: heart rates from 25 beats per minute to 200 beats per minute, heart rate variability (HRV) of 0ms to 200ms and arrhythmias, wherein the arrhythmias comprise at least one of: supraventricular tachycardia, and bradycardia.

4. The system (100) as claimed in claim 1, comprising at least one user device (102) comprises a user application configured to control operations of at least one of the at least one vital monitor (104) and the at least one ballistic micro-vibration validation device (106).

5. The system (100) as claimed in claim 1, wherein the user application in the at least one user device (102) enables a user to adjust at least one of intensity and duration of the micro-vibration generated by the at least one ballistic micro-vibration validation device (106).

6. The system (100) as claimed in claim 1, comprising a communication network (108) configured to connect at least one of the at least one user device (102), the at least one vital monitor (104), and the at least one ballistic micro-vibration validation device (106).

7. A method (600) for ballistic micro-vibration validation of vitals monitor, comprising:
evaluating at least one vital sign of at least one patient, by using at least one vital monitor (104);
simulating a mechanical micro-vibration of a heartbeat in a heart module (202); and
controlling and delivering power to the heart module (202), by using a microcontroller module (204); and
generating at least one micro-vibration for simulating at least one of various heart rates and arrhythmias for validating the at least one vital monitor (104), by using at least one ballistic micro-vibration validation device (106);
**characterized in that** the mechanical micro-vibration is simulated by using at least one eccentric rotating mass (302).

8. The method (600) as claimed in claim 7, comprising controlling operations of at least one of the at least one vital monitor (104) and the at least one ballistic micro-vibration validation device (106), by using a user application in at least one user device (102).

9. The method (600) as claimed in claim 7, comprising:
creating a lub-dub signal by using two short pulses in rapid succession;
setting an interval for repeating the lub-dub signal based on a desired heart rate by using the user device (102);
varying the interval based on a desired heart rate variability (HRV) and arrhythmia condition by using the user device (102);
repeating the lub-dub signal for every cardiac cycle by using the user device (102);
controlling the power and timing of the lub-dub signal precisely for simulating mechanical micro-vibrations of a heartbeat by using the microcontroller module (204);
simulating the mechanical micro-vibrations of the heartbeat by using the vibration module (302); and
validating the vital monitor (104) using a ballistic micro-vibration validation device (106).

10. The method (600) as claimed in claim 7, comprising enabling, by the at least one ballistic micro-vibration validation device (106) simulation of at least one of: heart rates from 25 beats per minute to 200 beats per minute, heart rate variability (HRV) of 0ms to 200ms and arrhythmias, wherein the arrhythmias comprises at least one of: supraventricular tachycardia, and bradycardia.

11. The method (600) as claimed in claim 7, comprising enabling a user to adjust at least one of intensity and duration of the micro-vibration generated by the at least one ballistic micro-vibration validation device (106), by using the user application in the at least one user device (102).

12. The method (600) as claimed in claim 7, comprising connecting at least one of the at least one user device (102), the at least one vital monitor (104), and the at least one ballistic micro-vibration validation device (106), by using a communication network (108).

## Patentansprüche

1. System (100) für Validierung ballistischer Mikrovibration eines Vitaldatenmonitors, umfassend:
mindestens einen Vitaldatenmonitor (104), der konfiguriert ist, um mindestens ein Vitalzeichen mindestens eines Patienten auszuwerten;
ein Herzmodul (202), das konfiguriert ist, um eine mechanische Mikrovibration eines Herzschlags zu simulieren;
ein Mikrocontrollermodul (204), das konfiguriert ist, um das Herzmodul (202) zu steuern und mit Leistung zu versorgen; und
mindestens eine Validierungsvorrichtung (106) für ballistische Mikrovibration, die konfiguriert ist, um mindestens eine Mikrovibration zum Simulieren mindestens eines von verschiedenen Herzfrequenzen und Arrhythmien zum Validieren des mindestens einen Vitaldatenmonitors (104) zu erzeugen;
**dadurch gekennzeichnet, dass** die mechanische Mikrovibration durch Verwenden mindestens einer exzentrischen rotierenden Masse (302) simuliert wird.

2. System (100) nach Anspruch 1, wobei der mindestens eine Vitaldatenmonitor (104) einen oder mehrere Sensoren zum Messen mindestens eines von Herzfrequenz, Blutsauerstoffsättigung und Blutdruck umfasst.

3. System (100) nach Anspruch 1, wobei die mindestens eine Validierungsvorrichtung (106) für ballistische Mikrovibration eine Simulation mindestens eines ermöglicht von: Herzfrequenzen von 25 Schlägen pro Minute bis 200 Schlägen pro Minute, Herzfrequenzvariabilität (HRV) von 0 ms bis 200 ms und Arrhythmien, wobei die Arrhythmien mindestens eine umfassen von: supraventrikuläre Tachykardie und Bradykardie.

4. System (100) nach Anspruch 1, umfassend mindestens eine Benutzervorrichtung (102), die eine Benutzeranwendung, die konfiguriert ist, um einen Betrieb mindestens eines von dem mindestens einen Vitaldatenmonitor (104) und der mindestens einen Validierungsvorrichtung (106) für ballistische Mikrovibration zu steuern, umfasst.

5. System (100) nach Anspruch 1, wobei die Benutzeranwendung in der mindestens einen Benutzervorrichtung (102) einem Benutzer ermöglicht, mindestens eine von Intensität und Dauer der Mikrovibration, die durch die mindestens eine Validierungsvorrichtung (106) für ballistische Mikrovibration erzeugt wird, anzupassen.

6. System (100) nach Anspruch 1, umfassend ein Kommunikationsnetzwerk (108), das konfiguriert ist, um mindestens eines von der mindestens einen Benutzervorrichtung (102), dem mindestens einen Vitaldatenmonitor (104) und der mindestens einen Validierungsvorrichtung (106) für ballistische Mikrovibration zu verbinden.

7. Verfahren (600) für Validierung ballistischer Mikrovibration des Vitaldatenmonitors, umfassend:
Auswerten mindestens eines Vitalzeichens mindestens eines Patienten durch Verwenden mindestens eines Vitaldatenmonitors (104);
Simulieren einer mechanischen Mikrovibration eines Herzschlags in einem Herzmodul (202); und
Steuern und Versorgen mit Leistung des Herzmoduls (202) durch Verwenden eines Mikrocontrollermoduls (204); und
Erzeugen mindestens einer Mikrovibration zum Simulieren mindestens eines von verschiedenen Herzfrequenzen und Arrhythmien zum Validieren des mindestens einen Vitaldatenmonitors (104) durch Verwenden mindestens einer Validierungsvorrichtung (106) für ballistische Mikrovibration;
**dadurch gekennzeichnet, dass** die mechanische Mikrovibration durch Verwenden mindestens einer exzentrischen rotierenden Masse (302) simuliert wird.

8. Verfahren (600) nach Anspruch 7, umfassend das Steuern des Betriebs mindestens eines von dem mindestens einen Vitaldatenmonitor (104) und der mindestens einen Validierungsvorrichtung (106) für ballistische Mikrovibration durch Verwenden einer Benutzeranwendung in mindestens einer Benutzervorrichtung (102).

9. Verfahren (600) nach Anspruch 7, umfassend:
Schaffen eines Lub-Dub-Signals durch Verwenden zweier kurzer Impulse in schneller Abfolge;
Einstellen eines Intervalls zum Wiederholen des Lub-Dub-Signals basierend auf einer gewünschten Herzfrequenz durch Verwenden der Benutzervorrichtung (102);
Variieren des Intervalls basierend auf einer gewünschten Herzfrequenzvariabilität (HRV) und einem Arrhythmiezustand durch Verwenden der Benutzervorrichtung (102);
Wiederholen des Lub-Dub-Signals für jeden Herzzyklus durch Verwenden der Benutzervorrichtung (102); präzises Steuern der Leistung und des Timings des Lub-Dub-Signals zum Simulieren mechanischer Mikrovibrationen eines Herzschlags durch Verwenden des Mikrocontrollermoduls (204);
Simulieren der mechanischen Mikrovibrationen des Herzschlags durch Verwenden des Vibrationsmoduls (302); und
Validieren des Vitaldatenmonitors (104) unter Verwendung einer Validierungsvorrichtung (106) für ballistische Mikrovibration.

10. Verfahren (600) nach Anspruch 7, umfassend das Ermöglichen, durch die mindestens eine Validierungsvorrichtung (106) für ballistische Mikrovibration, der Simulation mindestens eines von: Herzfrequenzen von 25 Schlägen pro Minute bis 200 Schlägen pro Minute, Herzfrequenzvariabilität (HRV) von 0 ms bis 200 ms und Arrhythmien, wobei die Arrhythmien mindestens eine umfassen von: supraventrikuläre Tachykardie und Bradykardie.

11. Verfahren (600) nach Anspruch 7, umfassend das Ermöglichen einem Benutzer, mindestens eine von Intensität und Dauer der Mikrovibration, die durch die mindestens eine Validierungsvorrichtung (106) für ballistische Mikrovibration erzeugt wird, durch Verwenden der Benutzeranwendung in der mindestens einen Benutzervorrichtung (102) anzupassen.

12. Verfahren (600) nach Anspruch 7, umfassend, das Verbinden mindestens eines von der mindestens einen Benutzervorrichtung (102), dem mindestens einen Vitaldatenmonitor (104) und der mindestens einen Validierungsvorrichtung (106) für ballistische Mikrovibration durch Verwenden eines Kommunikationsnetzwerks (108).

## Revendications

1. Système (100) de validation de micro-vibration balistique de surveillance de signes vitaux, comprenant :
au moins un moniteur vital (104) configuré pour évaluer au moins un signe vital d'au moins un patient ;
un module cardiaque (202) configuré pour simuler une micro-vibration mécanique d'un battement cardiaque ;
un module de microcontrôleur (204) configuré pour commander et fournir de l'énergie au module cardiaque (202) ; et
au moins un dispositif de validation de micro-vibrations balistiques (106) configuré pour générer au moins une micro-vibration pour simuler au moins l'une parmi diverses fréquences cardiaques et arythmies pour valider l'au moins un moniteur vital (104) ;
**caractérisé en ce que** la micro-vibration mécanique est simulée en utilisant au moins une masse rotative excentrique (302).

2. Système (100) selon la revendication 1, dans lequel l'au moins un moniteur vital (104) comprend un ou plusieurs capteurs pour mesurer au moins l'un parmi la fréquence cardiaque, la saturation en oxygène du sang et la pression artérielle.

3. Système (100) selon la revendication 1, dans lequel l'au moins un dispositif de validation des micro-vibrations balistiques (106) permet de simuler au moins l'un parmi : des fréquences cardiaques allant de 25 battements par minute à 200 battements par minute, une variabilité de la fréquence cardiaque (VFC) de 0 ms à 200 ms et des arythmies, dans lequel les arythmies comprennent au moins l'une parmi : une tachycardie supraventriculaire et une bradycardie.

4. Système (100) selon la revendication 1, comprenant au moins un dispositif utilisateur (102) comprenant une application utilisateur configurée pour commander les opérations d'au moins l'un parmi l'au moins un moniteur vital (104) et l'au moins un dispositif de validation de micro-vibrations balistiques (106).

5. Système (100) selon la revendication 1, dans lequel l'application utilisateur dans l'au moins un dispositif utilisateur (102) permet à un utilisateur de régler au moins l'une parmi l'intensité et la durée des micro-vibrations générées par l'au moins un dispositif de validation des micro-vibrations balistiques (106).

6. Système (100) selon la revendication 1, comprenant un réseau de communication (108) configuré pour connecter au moins l'un parmi l'au moins un dispositif utilisateur (102), l'au moins un moniteur vital (104), et l'au moins un dispositif de validation de micro-vibration balistique (106).

7. Procédé (600) de validation par micro-vibration balistique d'un moniteur de signes vitaux, comprenant :
l'évaluation d'au moins un signe vital d'au moins un patient, à l'aide d'au moins un moniteur vital (104) ;
la simulation d'une micro-vibration mécanique d'un battement de cœur dans un module cardiaque (202) ; et
la commande et la fourniture d'énergie au module cœur (202), à l'aide d'un module de microcontrôleur (204) ; et
la génération d'au moins une micro-vibration pour simuler au moins l'une parmi diverses fréquences cardiaques et arythmies afin de valider l'au moins un moniteur vital (104), en utilisant au moins un dispositif de validation de micro-vibration balistique (106) ;
**caractérisé en ce que** la micro-vibration mécanique est simulée en utilisant au moins une masse rotative excentrique (302).

8. Procédé (600) selon la revendication 7, comprenant la commande des opérations d'au moins l'un parmi l'au moins un moniteur vital (104) et l'au moins un dispositif de validation des micro-vibrations balistiques (106), en utilisant une application utilisateur dans au moins un dispositif utilisateur (102).

9. Procédé (600) selon la revendication 7, comprenant :
la création d'un signal lub-dub à l'aide de deux impulsions courtes en succession rapide ;
le réglage d'un intervalle de répétition du signal lub-dub en fonction d'une fréquence cardiaque souhaitée à l'aide du dispositif utilisateur (102) ;
la variation de l'intervalle en fonction d'une variabilité de la fréquence cardiaque (VFC) souhaitée et d'une condition d'arythmie à l'aide du dispositif utilisateur (102) ;
la répétition du signal lub-dub pour chaque cycle cardiaque à l'aide du dispositif utilisateur (102) ; la commande de la puissance et de la synchronisation du signal lub-dub avec précision pour simuler des micro-vibrations mécaniques d'un battement de coeur à l'aide du module de microcontrôleur (204) ;
la simulation des micro-vibrations mécaniques des battements cardiaques à l'aide du module de vibrations (302) ; et
la validation du moniteur vital (104) à l'aide d'un dispositif de validation des micro-vibrations balistiques (106).

10. Procédé (600) selon la revendication 7, comprenant l'activation, par l'au moins un dispositif de validation de micro-vibration balistique (106), de la simulation d'au moins l'un parmi : des fréquences cardiaques allant de 25 battements par minute à 200 battements par minute, une variabilité de la fréquence cardiaque (VFC) de 0 ms à 200 ms et des arythmies, dans lequel les arythmies comprennent au moins l'un parmi : une tachycardie supraventriculaire et une bradycardie.

11. Procédé (600) selon la revendication 7, comprenant le fait de permettre à un utilisateur de régler au moins l'une parmi l'intensité et la durée des micro-vibrations générées par l'au moins un dispositif de validation des micro-vibrations balistiques (106), en utilisant l'application utilisateur dans l'au moins un dispositif utilisateur (102).

12. Procédé (600) selon la revendication 7, comprenant la connexion d'au moins l'un parmi l'au moins un dispositif utilisateur (102), l'au moins un moniteur vital (104) et l'au moins un dispositif de validation de micro-vibrations balistiques (106), à l'aide d'un réseau de communication (108).
